# EUROPEAN PATENT SPECIFICATION

(11) **EP 3 805 272 B1**
(45) Date of publication and mention of the grant of the patent: **19.02.2025**
(21) Application number: 19864067.4
(22) Date of filing: 05.09.2019
(51) Int. Cl.: C08C 19/02, C08C 19/08, C08C 19/06, A61L 24/00

(54) **POLYHYDROXYL-CONTAINING POLYMER, PREPARATION METHOD THEREFOR AND USE THEREOF**
POLYHYDROXYLHALTIGES POLYMER, VERFAHREN ZU SEINER HERSTELLUNG UND SEINE VERWENDUNG
POLYMÈRE CONTENANT UN POLYHYDROXYLE, SON PROCÉDÉ DE PRÉPARATION ET UTILISATION ASSOCIÉE

(30) Priority: 29.09.2018 CN 201811152501
(43) Date of publication of application: 14.04.2021
(73) Proprietor: Nova Advanced Materials Co., Ltd, Denver, Colorado 080203 (US); Novashin Co., Ltd, Beijing 100085 (CN)
(72) Inventor: DAI, Junming, Beijing 100085 (CN); YANG, Jianchun, Beijing 100085 (CN); YANG, Dongmei, Beijing 100085 (CN); HAN, Kai, Beijing 100085 (CN); ZHU, Rongxin, Beijing 100085 (CN)
(74) Representative: Gille Hrabal Partnerschaftsgesellschaft mbB Patentanwälte
(86) International application number: PCT/CN2019/104592
(87) International publication number: WO 2020/063293

(56) References cited:
- EP-A1- 0 522 656
- EP-A1- 0 522 656
- EP-A2- 1 251 135
- CN-A- 1 297 443
- CN-A- 107 446 074
- US-A- 3 607 982
- US-A- 5 773 524
- DATABASE WPI Week 201833, Derwent World Patents Index; AN 2018-28114M, XP002804157

## Description

### Technical Field

The application belongs to the field of polymer materials which especially relates to a polyhydroxyl-containing polymer, its preparation method and application in vascular embolism.

### Background of the Invention

Interventional therapy is one of the fastest growing medical disciplines in the 21^{st} century. At present, interventional therapy keeps abreast with medical treatment and surgical treatment, and becomes the third treatment system. Embolization is an important part of interventional therapy, which is a minimal invasive treatment utilizing modern high-tech means. Embolization treatment is to inject an embolic body into the supply blood vessel of the lesion organ through intra-arterial or intravenous catheter under the guidance of a medical imaging equipment, to block the supply blood vessel and interrupt blood supply, in order to achieve the purpose of controlling bleeding, treating tumors and vascular lesions as well as eliminating the function of lesion organs.

Cerebral arteriovenous malformation (AVM) and aneurysm are the most common clinical hemorrhagic cerebrovascular diseases. They are prone to induce the epilepsy and twitch, nerve dysfunction and rupture hemorrhage, in which the disability rate and mortality rate are very high. Thus the embolism or blockage in the early stage becomes the best cure for such diseases.

The earlier way of embolizing cerebral aneurysm was surgical method which blocks the aneurysm already formed by cutting, ligature and clipping the neck of the aneurysm with the aneurysm clips, to block the impact of blood on the aneurysm. But this method has the disadvantages of time-consuming and possibility of rapture of the aneurysm.

In recent years, with the advancement of vascular imaging technology, the intravascular interventional therapy technology development is rapidly. Traditional surgery has been gradually replaced by intravascular embolization. This technology can provide patients with a treatment having the best nerve kinetic energy preservation, the best effect, maximum safety and minimal traumatic pain. It has gradually become an important part for the AVM and comprehensive treatment of aneurysm. Especially, the development of the microcatheter technology and guide wire technology has been able to approach blood vessels with a diameter as small as 1 mm, which can realize the treatment of multiple injuries in blood vessels.

Embolization can use the embolization method of platinum spring coil. US Patent Literature US6537293 and US6527790 described the application of electrolytic detachable spring coil and its auxiliary equipment in embolization treatment. The connection between the thruster of the electrolytic detachable coil and the spring coil adopts the micro welding technology. The coil is extremely soft so it puts low pressure on the aneurysm wall, and the position of the coil may be adjusted repeatedly, the thruster is connected to the positive electrode of the power supply to pass a weak current (0.5-2 mA), the stainless steel without insulation between the platinum coil and the thruster is electrolyzed and the spring coil can detach. However, the electrolytic detachable coil has defects such as incomplete embolization and low thrombosis rate due to unsatisfactory matching. In addition, it is prone to cause rupture and bleeding during the operation, and the manufacture process thereof is complicated and expensive. The electrolytic detachable spring coil is not an ideal embolization material.

In WO97/04657 and US Patent Literature US6476069 both had described the embolization technique by using the cyanoacrylate compounds as prepolymer. The cyanoacrylate compounds are a kind of liquid polymer monomer at room temperature, which is synthesized by the formaldehyde and the corresponding alkyl cyanoacetate and then the hydrolytic distillation. Due to the dual electron-withdrawing effect of cyano and carboxyl, the carbon atoms are catalyzed by anions in the blood and rapidly anionic polymerized, so as to function as a permanent embolism. Cyanoacrylate prepolymers liquid can be directly injected into a preset blood vessel through elongated microcatheter, so it is easy to operate. Relatively speaking, it is an ideal embolization material. But the existing cyanoacrylate embolization materials still have the disadvantage of "sticky to tube"; this shortcoming not only requires physicians to be skilled in operation because the microcatheter is extremely prone to adhere to the blood vessel wall after injection, resulting the operation failure. In addition, the monomer emits heat during monomer anionic polymerization, which is also another disadvantage of this embolization material.

Embolization microspheres are currently common embolization carriers, available materials of which include biodegradable materials such as gelatin and polylactic acid and non-biodegradable materials such as ethyl cellulose and polyvinyl alcohol (PVA). PVA is the most commonly used embolization material due to its nature such as safety, biocompatibility and permanent embolization. However, the efficacy of this microsphere embolization is limited by the interventional physician's personal intubation technique; different lesion locations have different requirements for microsphere particles, at the same time, the effect of the swelling rate and swelling pressure of the microspheres must also be considered. Therefore, there are many reports on embolization microspheres, but few products are actually used in the clinic.

WO97/04657 described a composition consists of ethylene vinyl alcohol polymer (EVOH) as biocompatible polymer, dimethyl sulfoxide as a solvent by adding biocompatible water-insoluble developer. The composition is released through a catheter to form an embolism at the preset vascular site.

The application US 3 607 982 A discloses hydroxylated block copolymers which are similar to the ones of the current invention. The polymers disclosed in said application deviate from the ones of the current invention as they do not comprise a unit according to formula 4 described below.

The application EP 0522656 A also discloses polymers which are similar to the ones of the current invention.

### Summary of the Invention

The catheter release technology which using water-insoluble biocompatible polymer as the main material for embolization can provide patients with a treatment having the best nerve kinetic energy preservation, the best effect, maximum safety and minimal traumatic pain. This technology has gradually become an important means for tumor treatment and injury treatment such as aneurysms, arteriovenous malformations (AVM), arteriovenous fistula (AVF), uncontrolled bleeding and its similar injuries.

The accurate release of the biocompatible polymer composition to the preset vascular site is the key to catheter release technology. For this reason, the embolization composition is added with a kind of water-insoluble and radiopaque developer. In this way, conventional techniques such as fluoroscopic examination can be used to observe the embolization process in real time. The use of water-insoluble developer is helpful to observe the embolism body in the subsequent treatment process (for example during surgery), or helpful to monitor the condition, and retreatment.

The embolic agent which added with therapeutic drugs (such as anti-tumor drugs, etc.) has dual function of embolism and targeted therapy. The treatment of specific tissues and organs has the advantages such as high targeting, good embolization effect, and the treatment can be combined with chemotherapeutics, magnetic fluids and radionuclides, also the treatment can sustained release drugs.

Among the currently available liquid embolic agents, the preferred one is polymer EVOH solution in dimethyl sulfoxide, the structure of EVOH is 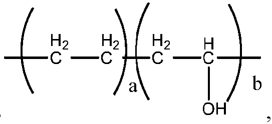 and it is a kind of alcoholysis products through the saponification or the partial saponification of ethylene-vinyl acetate (EVAc) polymer. EVOH is highly crystallized; in its structure, the ethylene content is 25% -40% (mole percent), it has good barrier properties, the gas permeability increases with increasing ethylene content. During the use of EVOH liquid embolic agent, the precipitated EVOH will still adhere to the orifice of the PE microcatheter, the bonding between the precipited mass blood vessel is not strong, the surgery requires a high level of physician's operation, moreover, the precipited mass is prone to stick to the microcatheter orifice, and this increases the risk of surgical failure.

For improving the deficiencies of the prior art, the object of the present invention is to provide a polyhydroxyl-containing polymer, and its preparation method and application. The so-called "polyhydroxyl", as shown in formula 1, means that each of two adjacent carbon atoms of the polymer backbone attaches one hydroxyl respectively, and optionally that a carbon atom of the polymer backbone attaches a hydroxyl and the polymer backbone contains a plurality of such structural units; the polymerization raw material of the polymer is a diene monomer, the diene monomer (for example, 1,3-butadiene monomers or isoprene) is self-polymerized and the polymer containing multiple double bonds represented by formula 5 is obtained, the polymer containing double bonds represented by formula 5 is oxidized to prepare an epoxidized polymer, the epoxidized polymer is catalytically hydrogenated and / or hydrolyzed to obtain polymers containing polyhydroxyl groups on the C-C chain. The polymer has good biocompatibility, at the same time, it has strong binding properties with the substance containing oxygen, nitrogen that is prone to form hydrogen bonds on surface, etc. The solution formed by the polymer of the present invention has poor adhesion to the intubation microcatheter, but it has strong adhesion to blood vessels, skin and glass, and thus can be used as a vascular embolic agent.

The object herein is achieved through the following technical solutions:

A polymer comprising the units of the following formula 1, formula 2, formula 3 and formula 4: 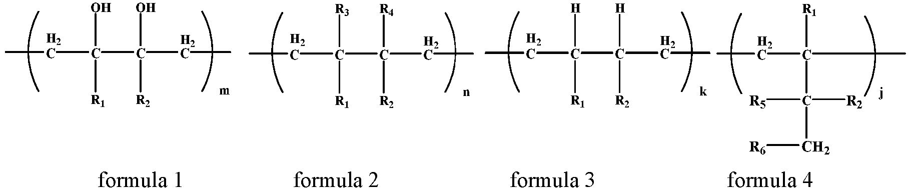 wherein, m is greater than or equal to 1, n, k is greater than or equal to 0, j>0; R₁, R₂ are the same or different, independently selected from H, at least one of C₁₋₈ alkanes; R₃ and R₄ are selected from H or hydroxyl and R₃ and R₄ are different, R₅ and R₆ are the same or different, independently selected from H or hydroxyl.

According to one aspect of the present invention, R₁ and R₂ are the same or different and are independently selected from H or methyl.

According to one aspect of the present invention, k=0.

According to the present invention, the polymer may be a homopolymer, a random copolymer or a block copolymer.

According to one aspect of the present invention, in the polymer, the number of the repeating unit represented by formula 1 is 0.1-100% of the total number of repeating units, the number of the repeating unit represented by formula 2 is 0-99.9% of the total number of repeating units, the number of the repeating unit represented by formula 3 is 0-99.9% of the total number of repeating units, the number of the repeating unit represented by formula 4 is 0-99.9% of the total number of repeating units.

Preferably, in the polymer, the number of the repeating unit represented by formula 3 is 0% of the total number of repeating units.

Preferably, in the polymer, the number of the repeating unit represented by formula 1 is 25-100% of the total number of repeating units, the number of the repeating unit represented by formula 2 is 0-75% of the total number of repeating units. ; further preferably, in the polymer, the number of the repeating unit represented by formula 1 is 50-100% of the total number of repeating units, the number of the repeating unit represented by formula 2 is 0-50% of the total number of repeating units; further preferably, in the polymer, the number of the repeating unit represented by formula 1 is 75-100% of the total number of repeating units, the number of the repeating unit represented by formula 2 is 0-25% of the total number of repeating units.

According to one aspect of the present invention, the number average molecular mass of the polymer is 10,000-220,000, preferably, the number average molecular mass of the polymer is 20,000-200,000, such as 50,000-200,000, such as 60,000-200,000.

According to the present invention, the polymer may be dissolved in organic solvents with good biocompatibility such as dimethyl sulfoxide, acetic acid and ethanol, etc.

The invention also provides a preparation method of the above polymer, the method comprises the following steps: 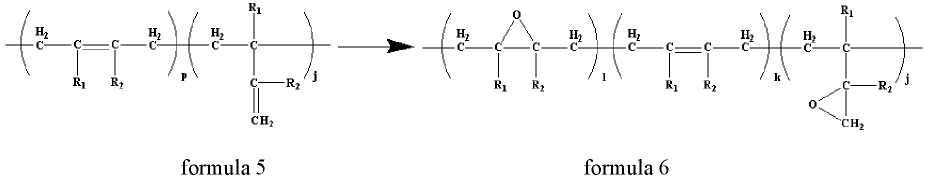
(1) the epoxidized polymer represented by formula 6 is prepared by oxidizing the polymer containing double bond represented by formula 5, wherein, R₁ and R₂ are defined as above, p=m+n+k, l=m+n, the definitions of j, k, m and n are as described above;
(2) then the epoxidized polymer represented by formula 6 is catalytically hydrogenated and / or hydrolyzed to prepare the polymer product.

According to the present invention, in step (1), the polymer represented by formula 5 may be obtained by self-polymerization of diene monomers; the diene monomer, for example, may be 1,3-butadiene or isoprene.

The preparation method of the polymer represented by formula 5 is as follows:
through a continuous solution polymerization, 1,3-butadiene or isoprene is mixed with alkanes, aromatics or a mixture of the both, such as toluene-heptane mixed solvent, under 30-65°C, into which is adding the initiator nickel naphthenate-BF₃-Et₃Al to initiate the reaction; octanol is added to adjust the molecular weight, and ethanol is added as a reaction terminator, the polymer represented by formula 5 was prepared.

According to the present invention, in step (1), the oxidation reaction includes but is not limited to chlorohydrin method, peroxide-epoxidation method or oxygen direct oxidation method.

Exemplarily, the peroxide may be one or more selected from hydrogen peroxide, peroxyformic acid, peroxyacetic acid and tert-butyl hydroperoxide, etc.

Exemplarily, the oxidation reaction may be carried out in an organic solution or a water / organic solvent emulsion of the polymer, the organic solvent includes but is not limited to fatty alkanes, halogenated aliphatic hydrocarbons, aromatic hydrocarbons, naphthenes, naphtha, etc., preferably hexane, cyclohexane, heptane, methylene chloride, benzene, toluene, naphtha, etc.

Exemplarily, the temperature of the oxidation reaction is 0 - 120 °C, preferably 20 - 80 °C.

According to the present invention, in step (2), the catalytic hydrogenation can open the epoxy ring of the epoxidized polymer by catalytic hydrogenation and the like to obtain polymers containing hydroxyl on the C-C chain; the hydrolysis can hydrolyze the epoxidized polymer by conventional acidic or basic substances, and open the epoxy ring to obtain the polymer containing adjacent dihydroxyl groups on the C-C chain.

The specific reaction conditions and material ratios of the catalytic hydrogenation and hydrolysis and the like described herein are all conventional choices in the art; there is no special limitation herein.

Exemplarily, the acidic substance includes inorganic acids such as aqueous hydrogen halide, sulfuric acid, nitric acid, etc.; organic acids such as alkyl sulfonic acid, etc.; solid acids and heteropoly acids, etc.

Exemplarily, the catalytic hydrogenation is catalyzed by raney nickel or platinum, palladium, etc.

Exemplarily, the catalytic hydrogenation reaction may be carried out in an organic solution or a water / organic solvent emulsion of the polymer, the organic solvent includes but is not limited to fatty alkanes, halogenated aliphatic hydrocarbons, naphthenes, naphtha, cyclic ether compounds, alcohols, etc., and is preferably selected from hexane, cyclohexane, tetrahydrofuran, methanol, ethanol, etc. The temperature of the catalytic hydrogenation reaction is 0 - 120 °C, preferably 20 - 80 °C.

Exemplarily, the hydrolysis reaction may be carried out in an organic solution or water / organic solvent emulsion of the polymer, the organic solvent includes but is not limited to aliphatic alkanes, halogenated aliphatic hydrocarbons, aromatic hydrocarbons, naphthenes, naphtha, cyclic ether compounds, sulfoxides, sulfones, pyrrolidone, methylpyrrolidone, etc., and is preferably selected from tetrahydrofuran, dimethyl sulfoxide, methylpyrrolidone, etc. The temperature of the hydrolysis reaction is -20 ~ 150 °C, preferably -10 ~ 80 °C.

The present invention further provides the use of the above polymer for vascular embolization.

### The Beneficial Effects of the Invention

The present invention provides a polyhydroxyl-containing polymer, preparation method and application thereof; the polymer has good biocompatibility, relatively strong binding properties with substances containing oxygen, nitrogen and that is prone to form hydrogen bonds, etc. The solution formed from the polymer of the present invention has poor adhesion to the intubation microcatheter, but it has strong adhesion to blood vessels, skin and glass, and thus can be used as a vascular embolic agent.

### Brief Description of the Drawing

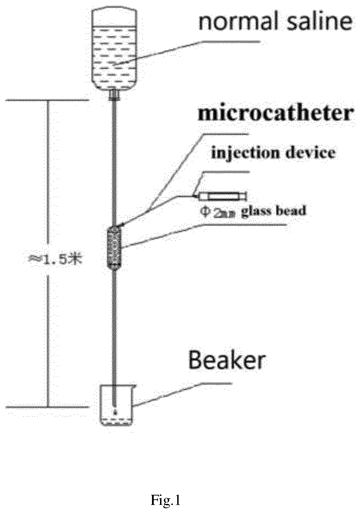

FIG. 1 is a schematic structural diagram of an in vitro experimental device for liquid embolic agent.

### Detailed Description of the Invention

The preparation method of the present invention will be further described in detail below in conjuction with specific examples. It should be understood that the following embodiments are merely illustrative and explanatory of the present invention; and should not to be construed as limiting the protection scope of the present invention. All technologies implemented based on the above contents of the present invention are covered by the scope of the present invention.

Unless otherwise specified, the experimental methods used in the following examples are conventional methods; unless otherwise specified, the reagents and materials used in the following examples can be obtained commercially.

The material structure tests ¹HNMR, ¹³CNMR employ the 600M pulse Fourier transform nuclear magnetic resonance spectrometers from JOEL company, IR is tested on Nicoletis 50, a Fourier infrared transform spectrometer from Thermofisher; the test of Tg values determined by using the Q100 Differential Scanning Calorimeter from TA company. The polymer molecular weight was tested on Agilent PL-GPC50 (equipped with differential refractive index detector and evaporative light scattering detector).

EVOH-1, EVOH-2 and EVOH-3 were all purchased from Kuraray, the differences among the three were the contents of ethylene. See the table in Test Example 1 for details.

### Example 1

cis-Polybutadiene: Commercially available polybutadiene mass (purchased from Sichuan Petrochemical Ltd., 1,4-cis content ≥98%, and 2% 1,4-trans and the structure represented by formula 4; number average molecular weight **M̅ₙ** = 170,000) were cut into approximately 2 mm mass particles.

Epoxidation: Into a 250ml three-neck flask equipped with a stirrer and thermometer, 7g of the above polybutadiene mass particles and 100 ml of methylene chloride was added, the mass was dissolved and stirred under constant temperature in water bath. Nitrogen was introduced for a few minutes when the mass was completely dissolved and became a sticky state. 9.90 g formic acid was added; 21.40 g of 30% aqueous hydrogen peroxide solution was added dropwise with stirring; the reaction temperature was held for about 15 h; according to ¹HNMR (CDCl₃) analysis, the characteristic chemical shift peaks of 1,4-cis double bond disappeared (the characteristic chemical shift of epoxy group δ=2.98, the characteristic chemical shift of 1,4-cis double bond δ=5.40). The reaction product was neutralized with 10% sodium carbonate solution to pH = 7, the water phase was seperated, the organic phase was washed and the phases were separated; absolute ethanol was added to the separated gel to precipitate, the precipitate was separated and washed with absolute ethanol once, waste liquid was filtered out to obtain a wet mass, which was dried at room temperature for 12 h, and then dried in a vacuum oven at 40 °C for about 24 hours to constant weight to obtain 7.3 g of epoxidized butadiene rubber.

Hydrolysis: 1 g of the epoxidized butadiene rubber prepared above was weighted and dissolved in 100 ml of tetrahydrofuran, into which a solution of 5 ml water and 1 ml perchloric acid was added dropwise with stirring at 25 °C. The addition was completed in 30 min and the stirring was continued at 25 °C for 12 hours. According to ¹HNMR (DMSO) analysis, the characteristic chemical shift peak of the epoxidized butadiene rubber disappeared (characteristic chemical shift of epoxy group δ=2.98, characteristic chemical shift of the adjacence dihydroxyl δ=4.18). Sodium carbonate was added into the reaction solution for neutralization. 1000 ml of water was added into the reaction solution dropwise to precipitate; the precipitate was separated, into which was added 400 ml of water and then soaked for 24 h. The water was filtered out. The resulting polymer substance was dried for 24 h at room temperature and then at 40 °C in a vacuum oven to constant weight. 1.12 g of solid matter was obtained. The molecular weight of the product M̅n=146,000, and Tg value thereof was 56 °C.

### Example 2

cis-Polybutadiene: 180 ml dry petroleum ether (60-90 °C) was added to a 500ml anhydrous and oxygen-free reaction flask, dry 1,3-butadiene (Chengdu Keyuan Gas Ltd., purity greater than or equal to 99.5%) was introduced at 5 °C, 20 g butadiene was absorbed. The reaction flask was sealed and put into a 40 °C water bath, 0.4 ml of aged liquid of nickel naphthenate and triisobutylaluminum was introduced with stirring, then 0.38 ml BF₃ diethyl ether complex and 0.4 ml n-octanol was introduced. The mixture was stirred and reacted for 2 h, 2 ml of ethanol was added to terminate the reaction. 200 ml of absolute ethanol was added to the reaction liquid to precipitate the solid mass. 19g of cis-Poly-1,4-butadiene was obtained after drying. GPC analysis of cis-polybutadiene indicated that the number average molecular weight was 91,000. Through the determination of HNMR, the cis-1,4 double bond ratio was 98.5%, gel content was less than 0.1%.
Epoxidation: The procedure was the same as that in Example 1.
Hydrolysis: The procedure was the same as that in Example 1.
1.1 g of solid product was obtained with a number average molecular weight of 72,000 and a Tg value of 48 °C.

### Example 3

cis-Polybutadiene: The steps were the same as those in Example 2, except that the polymerization temperature of 40 °C was replaced wiht 25 °C and the amount of n-octanol added was increased to 0.6ml. 17.8 g of cis-polybutadiene polymer was obtained, with a number average molecular weight of 23,000 and a cis-1,4 double bond ratio of 98.5%.
Epoxidation: The procedure was the same as that in Example 1.
Hydrolysis: The procedure was the same as that in Example 1.
1.1 g of solid matter was obtained with a number average molecular weight of 21,000 and a Tg value of 28 °C.

### Example 4

cis-Polybutadiene: The steps were the same as those in Example 2, except that the polymerization temperature of 40 °C was replaced with 30 °C and the amount of n-octanol added was 0.4 ml. The number average molecular weight of the polybutadiene polymer obtained was 50500, the cis-1,4 double bond ratio was 99%.
Epoxidation: The procedure was the same as that in Example 1.
Hydrolysis: The procedure was the same as that in Example 1
The number average molecular weight of the solid product obtained was 49,000, and its Tg value was 35 °C.

### Example 5

cis-Polybutadiene: The steps were the same as those in Example 2, except that the polymerization temperature of 40 °C was replaced with 45 °C and no n-octanol was added. The number average molecular weight of the polybutadiene polymer obtained was 250,000, the cis-1,4 double bond ratio was 99%.
Epoxidation: The procedure was the same as Example 1.
Hydrolysis: The procedure was the same as Example 1
The number average molecular weight of the solid product obtained was 245,000, and its Tg value was 72 °C.

### Example 6

2 g epoxidation product of Example 1 was dissolved in 200ml of freshly distilled tetrahydrofuran and then was added into a 500ml stainless steel autoclave, into which 0.4 g Raney nickel (immersed in ethanol, rinsed 3 times with tetrahydrofuran before being added into the reactor) was added. The nitrogen in the autoclave was charged to a pressure of 1 MPa and released to normal pressure. The nitrogen charging and releasing were repeated for 3 times. Hydrogen was introduced with stirring at 50 °C and was charged to a pressure of 1 MPa. The hydrogen pressure was kept and the reaction was stirred for 12 hours. According to ¹HNMR test, the degree of ring opening of epoxy group was about 75% (mol).

The temperature of the reaction liquid was reduced to 0 °C, and the pressure relief was conducted. The catalyst was filtered.

A solution formulated with 5 ml water, 1 ml perchloric acid and 5 ml tetrahydrofuran was added dropwise into the reaction liquid from which the catalyst had been filtered out. The addition was completed within 30 min, and the reaction temperature was allowed to rise to 25 °C. The temperature was maintained and stirring was conducted for 12 hours. According to ¹HNMR (DMSO) analysis, the characteristic chemical shift peak of the epoxidized butadiene rubber had disappeared; the units with adjacent dihydroxyl accounted for 24.5%. 0.37 g solid sodium carbonate was added into the reaction liquid and the mixture was stirred for 2 hours. Water was added dropwise into the reaction liquid to precipitate, to which was add with water and soaked for 24 h. Water was filtered. The resulting polymer substance was dried for 24 h at room temperature and dried to constant weight at 40 °C in a vacuum oven. 2.35g solid matter was obtained with a number average molecular weight of 133,000 and a Tg value of 66 °C.

### Example 7

10 g of the hydrolysate from Example 1 was added into 100ml DMSO. The mixture was stirred under nitrogen atmosphere at 50 °C for 12 h, a polymer solution was obtained. The polymer solution was placed into a container, sterilized and stored for further use.

The above polymer solution was introdued into nomal saline and precipitation occurred immediately. The precipitate gradually becomes firm and dense from the inside out. The experiment showed that the embolic composition provided by the present invention may rapidly cure, and form an elastic solid after curing.

**Table 1 The parameters of the polymers prepared in Examples 1-6**

| Example | Number average molecular weight / Ten thousand | Proportion%* of structural units of formula 1 | Proportion%* of structural units of formula 2 | Glass transition temperature /°C | Dimethyl sulfoxide dissolution |
|---|---|---|---|---|---|
| 1 | 14.6 | 100% | 0 | 56 | Heat to dissolve |
| 2 | 7.2 | 100% | 0 | 48 | Dissolve |
| 3 | 2.1 | 100% | 0 | 28 | Dissolve |
| 4 | 4.9 | 100% | 0 | 35 | Dissolve |
| 5 | 24.5 | 100% | 0 | 72 | Heat to swell |
| 6 | 13.3 | 24.5% | 75.5% | 66 | Dissolve |

| | | | | | |
|---|---|---|---|---|---|
| *Proportion of structural units of formula 1= moles of structural units of formula 1/ (moles of structural units of formula 1+ moles of structural units of formula 2) × 100%; Proportion of structural units of formula 2= moles of structural units of formula 2 / (moles of structural units of formula 1+ moles of structural units formula 2) × 100%. | | | | | |

As can be seen from the above examples 1-6, when the number average molecular weight was less than 50,000, the Tg value of the polymer was lower than 35 °C; When the molecular weight was higher than 240,000, it was hard to dissolve the polymer in solvents such as dimethyl sulfoxide, and it was difficult to formulate a solution.

### Test Example 1.

| Solution | Ethylene content, in mol% | adhesion at the outlet of micro-catheter | Whether there was embolic material flowing out during the process | Precipitated embolism mass | The adhesion of the precipitated embolism mass to the skin * |
|---|---|---|---|---|---|
| Example 1 | / | No adhesion | No | Elastic and sticky to glass rod | Adhesion |
| EVOH-1 | 27% | Adhesion | Yes | Elastic and not sticky to glass rod | No adhesion |
| EVOH-2 | 38% | Adhesion | No | Elastic and not sticky to glass rod | No adhesion |
| EVOH-3 | 48% | Adhesion | No | Elastic and not sticky to glass rod | No adhesion |
| Example 11 | / | No adhesion | No | Elastic and sticky to glass rod | Adhesion |

| | | | | | |
|---|---|---|---|---|---|
| * naked back skin of the rat after removing hair on back of the rats with electric hair removal scissors; the EVOH solution was prepared by referring to Example 7. | | | | | |

Test Example 1 was completed by using the following liquid embolic agent in vitro experimental device:
for a dropper of a disposable infusion set was filled with 2 mm diameter glass beads. The upper end of the dropper was connected to a normal saline bottle, the distance between the outlet of normal saline bottle and the bottom outlet of the hanging infusion set was greater than 1.5 m. The microcatheter was placed in the container having glass beads through a Y-shaped connector. The flow rate of normal saline at 37 °C was set to 0.3 ml/s. The liquid embolic agent was slowly injected into the dropper through the microcatheter via a constant velocity injection device; during the injection process, the change of flow rate of the normal saline was continuously measured. With the injection of embolic material, the water flow slows down until it was completely stopped. As for the embolic agent containing the compound in the experimental example, there was no embolic material flew out through the container of the glass beads during embolization. The embolic material was evenly dispersed in the glass bead container to achieve a good embolization effect. The precipitated embolic mass does not stick to the outlet of the microcatheter, so it confirmed that the compounds of the examples were compatible with different types of microcatheters.

### Test Example 2. Performance test of adhesion to glass

The polymer prepared in Examples 1, 4 and 6 and EVOH-1 (purchased from Kuraray, ethylene mol% was 27%) was added into the DMSO to dissolve and formulate into a solution with a concentration of 10%. PVA (purchased from Shanghai Chenqi Chemical Technology Ltd., the molecular weight was 80,000, the degree of alcoholysis was 99%) was dissolved in ethanol, so as to formulate a solution with a concentration of 10%.

The above polymer solutions were poured onto a clean extra clear glass surface respectively. The glass coated with EVOH-1, the polymer prepared in Examples 1, 4 and 6 were placed in a constant humidity space with humidity of approximately 90%; after 12 hours, the film surfaces were rinsed several times with water to rinse DMSO, then dried and the polymer films were peeled off. The glass coated with PVA-ethanol solution was dired directly and the polymer film was peeled off.

| | EVOH-1 | PVA | Example 1 | Example 4 | Example 6 |
|---|---|---|---|---|---|
| The difficulty of separation of polymer film from glass | Easy | Easy | Adhere together and cannot be peeled off | Adhere together and cannot be peeled off | Adhere together and cannot be peeled off |

Due to the adjacent dihydroxyl group, the polymer of the invention is prone to form strong hydrogen bonds with substances which contains oxygen, nitrogen and the like on the surface, and its adhesion to skin and glass is greater than that of EVOH and PVA. When applied in embolic agents, it not only has good precipitation of EVOH, but also greatly enhances the ability of clumpiness and reinforces the adhesion at the embolism site.

## Claims

1. A polymer **characterized in that**, the polymer comprises units represented by the following formula 1, formula 2, formula 3 and formula 4: 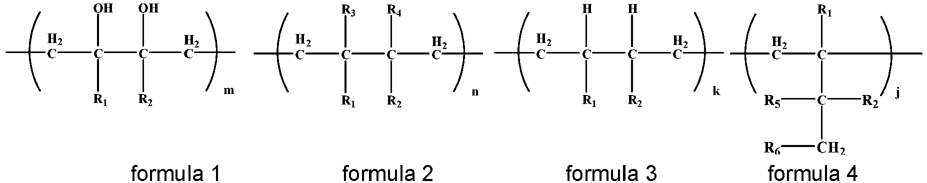 wherein, m is greater than or equal to 1, n, k is greater than or equal to 0; j>0; R₁, R₂ are the same or different, independently selected from H, at least one of C₁₋₈ alkanes; R₃ and R₄ are selected from H or hydroxyl and R₃ and R₄ are different, R₅ and R₆ are the same or different, independently selected from H or hydroxyl.

2. The polymer according to claim 1, **characterized in that**, the polymer is a homopolymer or a random copolymer or a block copolymer.

3. The polymer according to claim 1 or 2, **characterized in that**, in the polymer, the number of the repeating unit represented by formula 1 is 0.1 - lower than 100% of a total number of repeating units, the number of the repeating unit represented by formula 2 is 0-99.9% of the total number of repeating units, the number of the repeating unit represented by formula 3 is 0-99.9% of the total number of repeating units, the number of the repeating unit represented by formula 4 is greater than 0 - 99.9% of the total number of repeating units.

4. The polymer according to claim 3, **characterized in that**, in the polymer, the number of the repeating unit represented by formula 1 is 25 - lower than 100% of the total number of repeating units, the number of the repeating unit represented by formula 2 is 0-75% of the total number of repeating units.

5. The polymer according to claim 1 or 2, **characterized in that**, the number average molecular mass of the polymer is 10,000-220,000.

6. A preparation method of the polymer according to any one of claims 1 to 5, **characterized in that**, the method comprises the following steps: 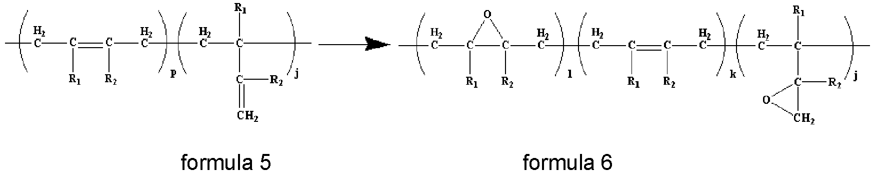
(1) the epoxidized polymer represented by formula 6 is prepared by oxidizing the polymer containing double bond represented by formula 5, wherein, R₁ and R₂ are defined as above, p=m+n+k, l=m+n, the definitions of j, k, m and n are as described above;
(2) then the epoxidized polymer represented by formula 6 is catalyticly hydrogenated and/or hydrolyzed to prepare the polymer product.

7. The preparation method according to claim 6, **characterized in that**, the polymer represented by formula 5 may be obtained by self-polymerization of diene monomers, the diene monomer may be, for example, 1,3-butadiene or isoprene.

8. The preparation method according to claim 6 or 7, **characterized in that**, in step (1), the oxidation reaction includes but is not limited to chlorohydrin method, peroxide-epoxidation method or oxygen direct oxidation method.

9. The preparation method according to any one of claims 6 to 8, **characterized in that**, in step (2), the catalytic hydrogenation can open the epoxy ring of the epoxidized polymer by catalytic hydrogenation to obtain polymers containing hydroxyl on the C-C chain; the hydrolysis can hydrolyze the epoxidized polymer by conventional acidic or basic substances, and open the epoxy ring to obtain the polymer containing adjacent dihydroxyl groups on the C-C chain.

10. A polymer according to any one of claims 1 to 5 for use in vascular embolization.

11. The polymer according to claim 1, **characterized in that**, R₁ and R₂ are the same or different and are independently selected from H or methyl.

12. The polymer according to claim 1, **characterized in that**, k=0.

13. The polymer according to claim 3, **characterized in that**, in the polymer, the number of the repeating unit represented by formula 3 is 0% of the total number of repeating units.

14. The polymer according to claim 3, **characterized in that**, the number of the repeating unit represented by formula 1 is 50-100% of the total number of repeating units, the number of the repeating unit represented by formula 2 is 0-50% of the total number of repeating units.

15. The polymer according to claim 3, **characterized in that**, the number of the repeating unit represented by formula 1 is 75-100% of the total number of repeating units, the number of the repeating unit represented by formula 2 is 0-25% of the total number of repeating units.

16. The preparation method according to claim 9, **characterized in that**, the acidic substance comprises inorganic acids such as aqueous hydrogen halide, sulfuric acid, nitric acid etc.; organic acids such as alkyl sulfonic acid etc.; solid acids and heteropoly acids, etc.

17. The preparation method according to claim 9, **characterized in that**, the catalytic hydrogenation is catalyzed by raney nickel, platinum, or palladium.

18. The preparation method according to claim 9, **characterized in that**, the catalytic hydrogenation reaction is carried out in an organic solution or a water / organic solvent emulsion of the polymer, the organic solvent includes fatty alkanes, halogenated aliphatic hydrocarbons, naphthenes, naphtha, cyclic ether compounds, alcohols, the temperature of the catalytic hydrogenation reaction is 0 - 120 °C.

19. The preparation method according to claim 9, **characterized in that**, the hydrolysis reaction is carried out in an organic solution of polymer or a water / organic solvent emulsion of the polymer, the organic solvent includes aliphatic alkanes, halogenated aliphatic hydrocarbons, aromatic hydrocarbons, naphthenes, naphtha, cyclic ether compounds, sulfoxides, sulfones, pyrrolidone, methylpyrrolidone, the temperature of the hydrolysis reaction is -20 ~ 150 °C.

## Patentansprüche

1. Polymer, **dadurch gekennzeichnet, dass** das Polymer Einheiten beinhaltet, die durch die folgende Formel 1, Formel 2, Formel 3 und Formel 4 dargestellt werden: 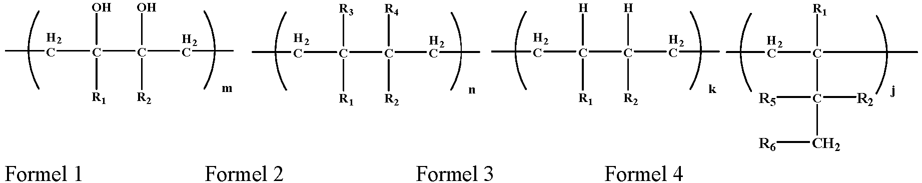 wobei m größer oder gleich 1 ist, n, k größer oder gleich 0 ist; j>0; R₁, R₂ gleich oder verschieden sind, unabhängig ausgewählt aus H, mindestens einem von C₁₋₈-Alkane, R₃ und R₄ ausgewählt sind aus H oder Hydroxyl und R₃ und R₄ unterschiedlich sind, R₅ und R₆ gleich oder verschieden sind, unabhängig ausgewählt aus H oder Hydroxyl.

2. Polymer nach Anspruch 1, **dadurch gekennzeichnet, dass** das Polymer ein Homopolymer oder ein statistisches Copolymer oder ein Blockcopolymer ist.

3. Polymer nach Anspruch 1 oder 2, **dadurch gekennzeichnet, dass** in dem Polymer die Anzahl der durch Formel 1 dargestellten Wiederholungseinheiten 0,1 - weniger als 100 % einer Gesamtanzahl der Wiederholungseinheiten beträgt, die Anzahl der durch Formel 2 dargestellten Wiederholungseinheiten 0 - 99,9 % der Gesamtanzahl der Wiederholungseinheiten beträgt, die Anzahl der durch Formel 3 dargestellten Wiederholungseinheiten 0 - 99,9 % der Gesamtanzahl der Wiederholungseinheiten beträgt und die Anzahl der durch Formel 4 dargestellten Wiederholungseinheiten größer als 0 - 99,9 % der Gesamtanzahl der Wiederholungseinheiten ist.

4. Polymer nach Anspruch 3, **dadurch gekennzeichnet, dass** in dem Polymer die Anzahl der durch Formel 1 dargestellten Wiederholungseinheiten 25 - weniger als 100 % der Gesamtanzahl der Wiederholungseinheiten beträgt, und die Anzahl der durch Formel 2 dargestellten Wiederholungseinheiten 0 - 75 % der Gesamtanzahl der Wiederholungseinheiten beträgt.

5. Polymer nach Anspruch 1 oder 2, **dadurch gekennzeichnet, dass** die zahlenmittlere Molekülmasse des Polymers 10.000 - 220.000 beträgt.

6. Herstellungsverfahren des Polymers nach einem der Ansprüche 1 bis 5, **dadurch gekennzeichnet, dass** das Verfahren die folgenden Schritte beinhaltet: 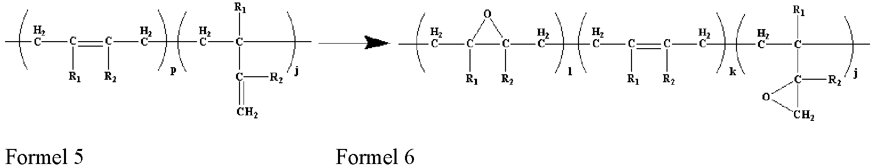
(1) das durch Formel 6 dargestellte epoxidierte Polymer wird durch Oxidation des durch Formel 5 dargestellten Polymers mit Doppelbindung hergestellt, wobei R₁ und R₂ wie oben definiert sind, p = m + n + k, l = m + n, die Definitionen von j, k, m und n wie oben beschrieben sind;
(2) anschließend wird das durch Formel 6 dargestellte epoxidierte Polymer katalytisch hydriert und/oder hydrolysiert, um das Polymerprodukt herzustellen.

7. Herstellungsverfahren nach Anspruch 6, **dadurch gekennzeichnet, dass** das durch Formel 5 dargestellte Polymer durch Selbstpolymerisation von Dienmonomeren erhalten werden kann, wobei das Dienmonomer beispielsweise 1,3-Butadien oder Isopren sein kann.

8. Herstellungsverfahren nach Anspruch 6 oder 7, **dadurch gekennzeichnet, dass** in Schritt (1) die Oxidationsreaktion das Chlorhydrin-Verfahren, das Peroxid-Epoxidierungsverfahren oder das Sauerstoff-Direktoxidationsverfahren umfasst, aber nicht darauf beschränkt ist.

9. Herstellungsverfahren nach einem der Ansprüche 6 bis 8, **dadurch gekennzeichnet, dass** in Schritt (2) die katalytische Hydrierung den Epoxidring des epoxidierten Polymers durch katalytische Hydrierung öffnen kann, um Polymere mit Hydroxyl an der CC-Kette zu erhalten; die Hydrolyse das epoxidierte Polymer durch herkömmliche saure oder basische Substanzen hydrolysieren und den Epoxidring öffnen kann, um das Polymer mit benachbarter Dihydroxylgruppen an der CC-Kette zu erhalten.

10. Polymer nach einem der Ansprüche 1 bis 5 zur Verwendung bei der Gefäßembolisierung.

11. Polymer nach Anspruch 1, **dadurch gekennzeichnet, dass** R₁ und R₂ gleich oder verschieden sind und unabhängig voneinander aus H oder Methyl ausgewählt werden.

12. Polymer nach Anspruch 1, **dadurch gekennzeichnet, dass** k = 0 ist.

13. Polymer nach Anspruch 3, **dadurch gekennzeichnet, dass** in dem Polymer die Anzahl der durch Formel 3 dargestellten Wiederholungseinheiten 0 % der Gesamtanzahl der Wiederholungseinheiten beträgt.

14. Polymer nach Anspruch 3, **dadurch gekennzeichnet, dass** die Anzahl der durch Formel 1 dargestellten Wiederholungseinheiten 50 - 100 % der Gesamtanzahl der Wiederholungseinheiten beträgt und die Anzahl der durch Formel 2 dargestellten Wiederholungseinheiten 0 - 50 % der Gesamtanzahl der Wiederholungseinheiten beträgt.

15. Polymer nach Anspruch 3, **dadurch gekennzeichnet, dass** die Anzahl der durch Formel 1 dargestellten Wiederholungseinheiten 75 - 100 % der Gesamtanzahl der Wiederholungseinheiten beträgt und die Anzahl der durch Formel 2 dargestellten Wiederholungseinheiten 0 - 25 % der Gesamtanzahl der Wiederholungseinheiten beträgt.

16. Herstellungsverfahren nach Anspruch 9, **dadurch gekennzeichnet, dass** die saure Substanz anorganische Säuren wie wässrigen Halogenwasserstoff, Schwefelsäure, Salpetersäure usw.; organische Säuren wie Alkylsulfonsäure usw.; feste Säuren und Heteropolysäuren usw. beinhaltet.

17. Herstellungsverfahren nach Anspruch 9, **dadurch gekennzeichnet, dass** die katalytische Hydrierung durch Raney-Nickel, Platin oder Palladium katalysiert wird.

18. Herstellungsverfahren nach Anspruch 9, **dadurch gekennzeichnet, dass** die katalytische Hydrierungsreaktion in einer organischen Lösung oder einer Wasser/organischen Lösungsmittelemulsion des Polymers durchgeführt wird, wobei das organische Lösungsmittel Fettsäurealkane, halogenierte aliphatische Kohlenwasserstoffe, Naphthene, Naphtha, zyklische Etherverbindungen, Alkohole umfasst, wobei die Temperatur der katalytischen Hydrierungsreaktion 0 - 120 °C beträgt.

19. Herstellungsverfahren nach Anspruch 9, **dadurch gekennzeichnet, dass** die Hydrolysereaktion in einer organischen Lösung des Polymers oder einer Wasser/organischen Lösungsmittelemulsion des Polymers durchgeführt wird, wobei das organische Lösungsmittel aliphatische Alkane, halogenierte aliphatische Kohlenwasserstoffe, aromatische Kohlenwasserstoffe, Naphthene, Naphtha, zyklische Etherverbindungen, Sulfoxide, Sulfone, Pyrrolidon, Methylpyrrolidon umfasst, wobei die Temperatur der Hydrolysereaktion -20 - 150 °C beträgt.

## Revendications

1. Polymère **caractérisé en ce que** le polymère comprend des unités représentées par la formule 1, la formule 2, la formule 3 et la formule 4 suivantes : 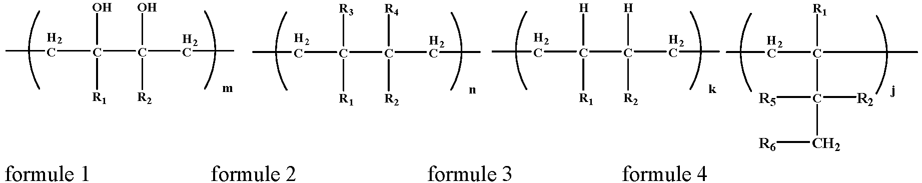 dans laquelle, m est supérieur ou égal à 1, n, k est supérieur ou égal à 0 ; j>0 ; R₁, R₂ sont identiques ou différents, choisis indépendamment parmi H, au moins un des C₁₋₈ alcanes ; R₃ et R₄ sont choisis parmi H ou hydroxyle et R₃ et R₄ sont différents, R₅ et R₆ sont identiques ou différents, choisis indépendamment parmi H ou hydroxyle.

2. Polymère selon la revendication 1, **caractérisé en ce que**, le polymère est un homopolymère ou un copolymère statistique ou un copolymère séquencé.

3. Polymère selon la revendication 1 ou 2, **caractérisé en ce que**, dans le polymère, le nombre d'unités répétitives représentées par la formule 1 est de 0,1 à moins de 100 % du nombre total d'unités répétitives, le nombre d'unités répétitives représentées par la formule 2 est de 0 à 99,9 % du nombre total d'unités répétitives, le nombre d'unités répétitives représentées par la formule 3 est de 0 à 99,9 % du nombre total d'unités répétitives, le nombre d'unités répétitives représentées par la formule 4 est supérieur à 0 à 99,9 % du nombre total d'unités répétitives.

4. Polymère selon la revendication 3, **caractérisé en ce que**, dans le polymère, le nombre d'unités répétitives représentées par la formule 1 est de 25 à moins de 100 % du nombre total d'unités répétitives, le nombre d'unités répétitives représentées par la formule 2 est de 0 à 75 % du nombre total d'unités répétitives.

5. Polymère selon la revendication 1 ou 2, **caractérisé en ce que**, la masse moléculaire moyenne en nombre du polymère est de 10 000 à 220 000.

6. Procédé de préparation du polymère selon l'une quelconque des revendications 1 à 5, **caractérisé en ce que**, le procédé comprend les étapes suivantes : 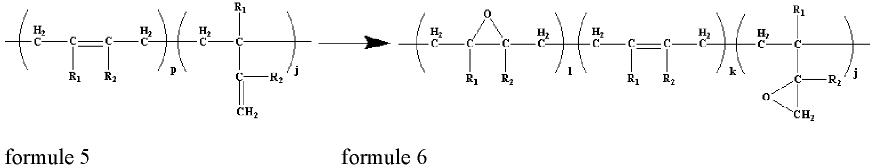
(1) le polymère époxydé représenté par la formule 6 est préparé en oxydant le polymère contenant une double liaison représenté par la formule 5, dans laquelle, R₁ et R₂ sont définis comme ci-dessus, p=m+n+k, l=m+n, les définitions de j, k, m et n sont telles que décrites ci-dessus ;
(2) ensuite, le polymère époxydé représenté par la formule 6 est hydrogéné et/ou hydrolysé de manière catalytique pour préparer le produit polymère.

7. Procédé de préparation selon la revendication 6, **caractérisé en ce que**, le polymère représenté par la formule 5 peut être obtenu par autopolymérisation de monomères diènes, le monomère diène pouvant être par exemple le 1,3-butadiène ou l'isoprène.

8. Procédé de préparation selon la revendication 6 ou 7, **caractérisé en ce que**, dans l'étape (1), la réaction d'oxydation comprend, sans s'y limiter, le procédé à la chlorhydrine, le procédé d'époxydation au peroxyde ou le procédé d'oxydation directe à l'oxygène.

9. Procédé de préparation selon l'une quelconque des revendications 6 à 8, **caractérisé en ce que**, dans l'étape (2), l'hydrogénation catalytique peut ouvrir le cycle époxy du polymère époxydé par hydrogénation catalytique pour obtenir des polymères contenant des groupes hydroxyle sur la chaîne C-C ; l'hydrolyse peut hydrolyser le polymère époxydé par des substances acides ou basiques classiques, et ouvrir le cycle époxy pour obtenir le polymère contenant des groupes dihydroxyle adjacents sur la chaîne C-C.

10. Polymère selon l'une quelconque des revendications 1 à 5 destiné à être utilisé dans l'embolisation vasculaire.

11. Polymère selon la revendication 1, **caractérisé en ce que**, R₁ et R₂ sont identiques ou différents et sont choisis indépendamment parmi H ou méthyle.

12. Polymère selon la revendication 1, **caractérisé en ce que**, k=0.

13. Polymère selon la revendication 3, **caractérisé en ce que**, dans le polymère, le nombre d'unités répétitives représentées par la formule 3 est de 0 % du nombre total d'unités répétitives.

14. Polymère selon la revendication 3, **caractérisé en ce que**, le nombre d'unités répétitives représentées par la formule 1 est de 50 à 100 % du nombre total d'unités répétitives, le nombre d'unités répétitives représentées par la formule 2 est de 0 à 50 % du nombre total d'unités répétitives.

15. Polymère selon la revendication 3, **caractérisé en ce que**, le nombre d'unités répétitives représentées par la formule 1 est de 75 à 100 % du nombre total d'unités répétitives, le nombre d'unités répétitives représentées par la formule 2 est de 0 à 25 % du nombre total d'unités répétitives.

16. Procédé de préparation selon la revendication 9, **caractérisé en ce que**, la substance acide comprend des acides inorganiques tels que l'halogénure d'hydrogène aqueux, l'acide sulfurique, l'acide nitrique, etc. ; des acides organiques tels que l'acide alkylsulfonique, etc. ; des acides solides et des hétéropolyacides, etc.

17. Procédé de préparation selon la revendication 9, **caractérisé en ce que**, l'hydrogénation catalytique est catalysée par du nickel de Raney, du platine ou du palladium.

18. Procédé de préparation selon la revendication 9, **caractérisé en ce que**, la réaction d'hydrogénation catalytique est réalisée dans une solution organique ou une émulsion eau/solvant organique du polymère, le solvant organique comprend des alcanes gras, des hydrocarbures aliphatiques halogénés, des naphtènes, du naphta, des composés éther cycliques, des alcools, la température de la réaction d'hydrogénation catalytique est de 0 à 120 °C.

19. Procédé de préparation selon la revendication 9, **caractérisé en ce que** la réaction d'hydrolyse est réalisée dans une solution organique de polymère ou une émulsion eau/solvant organique du polymère, le solvant organique comprend des alcanes aliphatiques, des hydrocarbures aliphatiques halogénés, des hydrocarbures aromatiques, des naphtènes, du naphta, des composés éther cycliques, des sulfoxydes, des sulfones, de la pyrrolidone, de la méthylpyrrolidone, la température de la réaction d'hydrolyse est de -20 à 150 °C.
